# FASCICULE DE BREVET EUROPEEN

(11) **EP 1 979 450 B1**
(45) Date de publication et mention de la délivrance du brevet: **22.06.2016**
(21) Numéro de dépôt: 06830716.4
(22) Date de dépôt: 19.12.2006
(51) Int. Cl.: A23D 9/007, B01D 11/04, C11B 1/10, C11B 7/00, C11C 1/00, C11C 1/08, C11C 3/00

(54) **PROCEDE D'ENRICHISSEMENT EN DHA**
DHA-ANREICHERUNGSVERFAHREN
DHA ENRICHMENT PROCESS

(30) Priorité: 20.12.2005 FR 0512933
(43) Date de publication de la demande: 15.10.2008
(73) Titulaire: PIERRE FABRE MEDICAMENT, 92100 Boulogne-Billancourt (FR)
(72) Inventeur: MARCIACQ, Florence, F-81600 Brens (FR); SOULAYRES, Mathieu, Québec, G1R 1S4 (CA); FREISS, Bernard, F-81100 Castres (FR)
(74) Mandataire: Regimbeau
(86) Numéro de dépôt international: PCT/EP2006/069899
(87) Numéro de publication internationale: WO 2007/071670

(56) Documents cités:
- EP-A- 0 712 651
- EP-A- 1 419 780
- EP-A1- 0 379 963
- WO-A-2004/028470
- FLECK U ET AL: "Fractionation of fatty acid ethyl esters by supercritical CO2: high separation efficiency using an automated countercurrent column" JOURNAL OF SUPERCRITICAL FLUIDS, PRA PRESS, US, vol. 14, no. 1, 1 octobre 1998 (1998-10-01), pages 67-74, XP004262488 ISSN: 0896-8446
- LUCIEN F P; LIONG K K; COTTON N J; MACNAUGHTON S J; FOSTER N R: "Separation of biomolecules using supercritical fluid extraction" AUSTRALASIAN BIOTECHNOLOGY., vol. 3, no. 3, 1993, pages 143-147, XP008066718 AUAUSTRALIAN BIOTECHNOLOGY ASS., CLAYTON. cité dans la demande
- MISHRA V K; TEMELLI F; OORAIKUL B: "Extraction and purification of omega-3 fatty acids with an emphasis on supercritical fluid extraction: A review" FOOD RESEARCH INTERNATIONAL., vol. 26, no. 3, 1993, pages 217-226, XP008066737 GBELSEVIER APPLIED SCIENCE, BARKING.
- RIHA V ET AL: "Separation of fish oil ethyl esters with supercritical carbon dioxide" JOURNAL OF SUPERCRITICAL FLUIDS, PRA PRESS, US, vol. 17, no. 1, 29 février 2000 (2000-02-29), pages 55-64, XP004262567 ISSN: 0896-8446
- ROBINSON D ET AL: "SUPPRESSION OF AUTOIMUUNE DISEASE BY DIETARY N-3 FATTY ACIDS", JOURNAL OF LIPID RESEARCH, AMERICAN SOCIETY FOR BIOCHEMISTRY AND MOLECULAR BIOLOGY, INC, US, vol. 34, no. 8, 1 August 1993 (1993-08-01) , pages 1435-1444, XP000610823, ISSN: 0022-2275
- NILSSON W B GAUGLITZ E J HUDSON J K: "Supercritical fluid fractionation of fish oil esters using incremental pressure programming and a temperature gradient", JOURNAL OF THE AMERICAN OIL CHEMISTS' SOCIETY, SPRINGER, DE, vol. 66, no. 11, 1 November 1989 (1989-11-01), pages 1596-1600, XP002954047, ISSN: 0003-021X, DOI: 10.1007/BF02636184

## Description

La présente invention concerne un procédé permettant, industriellement, d'enrichir une solution d'esters éthyliques d'acides gras naturels (huile de poisson) en un acide gras spécifique : l'acide docosahéxénoïque (couramment appelé DHA).

Les huiles d'origine marine sont des sources naturellement riches en acides gras poly-insaturés. Les acides gras polyinsaturés ont en particulier, pour les plus utiles les formules suivantes :

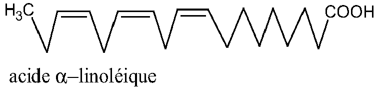

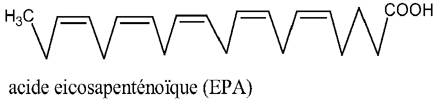

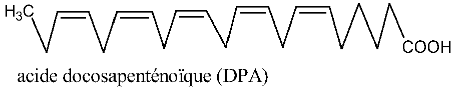

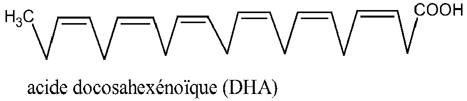

Ces acides gras, bien que nécessaires au bon fonctionnement de l'organisme, ne sont pas naturellement synthétisés par le corps humain. Leur apport est donc lié à une prise quotidienne via l'alimentation. Les sources alimentaires en acides gras poly-insaturés sont les huiles végétales (essentiellement des acides gras de type ω-6 et ω-9) et les huiles de poisson qui contiennent en particulier de grandes quantités d'acides gras de type ω-3. Ces derniers sont très connus pour leurs effets bénéfiques sur la santé (maladies cardiovasculaires, maladies auto-immunes, inflammations ...). Les acides gras polyinsaturés sont classés selon la position de la première double liaison, à partir de la fonction méthyle terminale. Ainsi dans la nomenclature ω-x ou n-x, le x correspond à la position de cette première insaturation. La majorité des acides gras polyinsaturés d'intérêt biologique appartient à la famille des ω-6 (acide arachidonique) ou ω-3 (EPA, DHA). De plus, dans la nomenclature, on définit également le nombre de carbone constituant la chaîne ; ainsi l'EPA est décrit comme C20:5 et le DHA comme C22:6. Le 5 et le 6 correspondent au nombre d'insaturations de la chaîne carbonée présenté respectivement par l'EPA et le DHA. Les huiles de poisson sont utilisées essentiellement pour l'isolement et la concentration en acides gras de type ω-3.

Les méthodes conventionnelles pour enrichir les huiles de poisson (article de V.K. Mishra et al., Food Research International, 1993, 26, 217-226) reposent sur la sélectivité par rapport à la longueur des chaînes des acides gras constitutifs des huiles ou leur degré d'insaturation. Les procédés d'enrichissement les plus couramment utilisés sont réalisés sur les acides gras ou les esters correspondants par :
- Cristallisation
- Extraction à contre-courant
- Distillation moléculaire
- Chromatographie d'absorption.
La plupart du temps, différents procédés sont combinés en vue d'obtenir un fort enrichissement. De plus ces procédés possèdent les inconvénients suivants :
Les procédés travaillant à haute température (distillation) donnent lieu à de multiples produits de dégradation thermique des acides gras (isomérisation, péroxydation, oligomérisation). De ce fait, il est recommandé de travailler à basse température, avantageusement à des températures inférieures à 100°C.
L'inconvénient des techniques de chromatographie repose sur l'utilisation de quantités massives de solvants souvent toxiques. De plus, la production à grande échelle sur de telles techniques est loin d'être évidente.

Pour ces raisons, des méthodes alternatives ont été développées. Elles reposent sur l'utilisation des fluides supercritiques :
- Procédé de fractionnement par CO2 supercritique
- Chromatographie supercritique.

Le procédé de fractionnement d'esters éthyliques d'acides gras par CO₂ supercritique a déjà été décrit abondamment dans la littérature. Il est à noter toutefois que la plupart des procédés cités décrivent un enrichissement en ω-3 ou en acide eicosapanténoïque (EPA) et non en DHA.
L'un des paramètres de conduite du procédé de fractionnement par CO₂ supercritique est le taux de CO₂ supercritique. Ce taux est défini par le rapport du débit de CO₂ sur le débit de solution d'acides gras injectée. Ainsi, à taux de CO₂ supercritique élevé, la sélectivité est accrue au détriment du rendement. A faible taux de CO₂ supercritique, c'est le rendement qui est favorisé tandis que la sélectivité est amoindrie.

Ainsi, Nilsson et al. ( JAOCS 1988, 65(1), 109-117) décrivent un procédé batch permettant d'obtenir plusieurs fractions (de 0,1 à 0,2g) riches en EPA et DHA respectivement. Pour ce faire, les auteurs travaillent à des pressions comprises entre 220*10⁵ Pa et 250 *10⁵ Pa. De plus, ils effectuent un gradient de température dans la colonne pour générer un reflux interne (de 20°C en pied de colonne à 100°C en tête de colonne). Les taux de CO₂ supercritique définis sont très élevés, de l'ordre de 100 à 500.
Certains essais permettent d'obtenir, à partir d'esters éthyliques pré-traités à l'urée, des fractions présentant une teneur de l'ordre de 90% en DHA, mais avec des taux de CO₂ supercritique de 500.
En utilisant des esters éthyliques non traités, les auteurs décrivent des fractions riches en DHA (teneur entre 53 et 60%) mais pour des taux de CO₂ supercritique de l'ordre de 300 à 400.
Ce procédé a donc les inconvénients suivants : il s'agit d'un procédé batch. Le taux de CO₂ supercritique utilisé est trop important et le procédé doit donc avoir un faible rendement, ce qui n'est pas utilisable industriellement. En effet, cela induit des surcoûts énergétiques et donc une productivité moindre.La température de 100°C en tête de colonne peut provoquer la dégradation des acides gras. Or il s'agit de la température préconisée par les auteurs. Les pressions utilisées sont trop fortes et si elles étaient diminuées, il y aurait une augmentation du taux de CO₂ supercritique. Par ailleurs cet article préconise l'utilisation d'un gradient de température au sein de la colonne et surtout d'un reflux interne afin d'améliorer la séparation et donc l'enrichissement. Or la présence d'un tel reflux diminue la productivité du procédé.

Kado et al. (JP2005-255971) décrivent dans leur brevet un procédé d'enrichissement d'esters éthyliques d'huile de poisson en EPA et DHA. Les plages de température et pression revendiquées sont de 35-200°C et 100*10⁵ Pa-500*10⁵ Pa. Les auteurs préconisent deux extractions successives en vue d'obtenir des teneurs élevées. Une première extraction est faite sur la matière première, et une deuxième extraction est faite sur le résidu de la première opération. La colonne utilisée fait 3m de haut pour un diamètre interne de 50mm. Elle comporte 6 enceintes de chauffe distinctes. Les deux extractions successives compliquent le procédé et le rendent inapplicable industriellement.
Les taux de CO₂ supercritique utilisés pour obtenir des teneurs élevées en DHA sont élevés (de l'ordre de 127). Le rendement en DHA est donc faible. Par ailleurs les auteurs préconisent l'application d'un gradient de température (qui permet d'avoir un effet de rectification) dans la colonne. La productivité est donc restreinte.

Lucien et col. (Australasian biotechnology, 1993, 3 (3), 143-147) décrivent un procédé d'obtention d'extraits enrichis en esters éthyliques d'EPA et de DHA à partir d'huile de sardine 17/12 (EPA/DHA). Un reflux interne généré par une enceinte chauffante placée en tête de colonne permet d'améliorer les teneurs obtenues : on peut atteindre des teneurs de 42% et 54% respectivement en EPA et DHA. Les meilleures conditions opératoires dans cette configuration sont 150 *10⁵ Pa, 40°C dans la colonne et une température de reflux de 100°C. Le taux de CO₂ supercritique utilisé n'est pas indiqué.
Le tableau ci-dessous reprend les résultats obtenus :

| Pression (Pa) | Température extraction (°C) | Température reflux (°C) | [EPA] (%) | [DHA] (%) |
|---|---|---|---|---|
| 150 *10⁵ | 40 | 60 | 21 | 34 |
| 150*10⁵ | 40 | 80 | 33 | 39 |
| 150*10⁵ | 40 | 100 | 42 | 54 |

Ce procédé a donc les inconvénients suivants : La température de 100°C en tête de colonne peut provoquer la dégradation des acides gras. Or il s'agit de la température préconisée par les auteurs. Par ailleurs cet article préconise l'utilisation d'un gradient de température au sein de la colonne et surtout d'un reflux interne afin d'améliorer la séparation et donc l'enrichissement. Or la présence d'un tel reflux diminue la productivité du procedé.

Fleck et al. (Journal of Supercritical Fluids, 1998, 14 (1), 67-74) décrivent un procédé d'enrichissement d'esters éthyliques d'huile de poisson en EPA et DHA, à contre-courant dans une colonne de fractionnement à l'aide de CO₂ supercritique. Les conditions opératoires sont 5 60°C, 145*10⁵ Pa et le taux de CO₂supercritique est de l'ordre de 255.

Enfin Zhu et col. (Proceedings of the 3rd International Symposium on supercritical fluids - Strasbourg, 1994) décrivent un procédé d'extraction /fractionnement d'une solution d'esters éthyliques d'acides gras en vue d'obtenir des fractions riches respectivement en EPA et en DHA. Pour ce faire, un flux de CO₂ extrait les esters d'acides gras contenus dans un extracteur, et ce flux de CO₂ chargé en esters est ensuite fractionné dans une colonne. Un gradient de température et une programmation de pression est effectuée au sein de la colonne, afin d'améliorer la sélectivité par rapport aux composés recherchés. Ce procédé batch permet d'isoler une fraction représentant 12% du DHA engagé avec une pureté supérieure à 50%. Ce procédé a donc les inconvénients suivants : il s'agit d'un procédé batch et c'est pour cela qu'il peut comprendre une programmation en pression. Le taux de CO₂ supercritique utilisé est trop important (211) et le procédé doit donc avoir un faible rendement, ce qui n'est pas utilisable industriellement. En effet, cela induit des surcoûts énergétiques et donc une productivité moindre. Par ailleurs cet article préconise l'utilisation d'un gradient de température au sein de la colonne et surtout d'un reflux interne afin d'améliorer la séparation et donc l'enrichissement. Or la présence d'un tel reflux diminue la productivité du procédé.

La majorité des procédés décrits dans la littérature utilisent donc un fort taux de CO₂ supercritique, ce qui donne un très faible rendement en DHA et donc un procédé qui n'est pas réalisable industriellement. De plus, afin qu'un procédé soit industriellement réalisable, il est également nécessaire de ne pas travailler à trop forte pression. Or, lorsque la pression diminue, la densité diminue et il faut donc augmenter le taux de CO₂ supercritique pour obtenir une teneur en DHA équivalente. Il convient donc de trouver un compromis entre pression, taux de CO₂ supercritique et rendement, afin d'obtenir un enrichissement intéressant en DHA, c'est à dire d'au moins 50%. Par ailleurs, seuls les procédés continus sont intéressants du point de vue industriel.
Enfin, afin d'éviter la dégradation des acides gras et en particulier du DHA, il convient de travailler à faible température, c'est à dire à une température inférieure à 100°C et en particulier inférieure ou égale à 70°C.
De plus, il convient également de trouver un procédé permettant d'enrichir une solution d'acides gras en DHA n'utilisant qu'une seule étape de fractionnement à contre-courant et ne nécessitant pas le pré-traitement des acides gras à l'urée.

Les inventeurs ont ainsi découvert de façon surprenante qu'une température d'au plus 70°C, qu'une pression comprise entre 100*10⁵ Pa et 160 *10⁵ Pa et qu'un taux de CO₂ supercritique compris entre 30 et 70 permettait, dans le cadre d'un procédé continu en une seule étape de fractionnement à contre-courant par CO₂ supercritique, d'obtenir un enrichissement en DHA d'au moins 50%, en partant d'une solution d'acides gras ou de ses dérivés comprenant moins de 50% de DHA par rapport aux acides gras totaux de la solution ou à leurs dérivés et n'ayant pas été prétraitée à l'urée.

La présente invention concerne donc un procédé continu d'enrichissement en DHA d'une solution d'acides gras ou de ses dérivés comprenant moins de 50% de DHA par rapport aux acides gras totaux de la solution ou à leurs dérivés, le procédé comprenant les étapes de
(a)- injection simultanée à contre-courant dans une colonne de fractionnement du flux de la solution d'acides gras ou de ses dérivés et d'un flux de CO₂ supercritique à une température inférieure ou égale à 70°C et à une pression comprise entre 100*10⁵ Pa et 160* 10⁵ Pa, avantageusement entre 120* 10⁵ Pa et 140* 10⁵ Pa, le taux de CO₂ supercritique étant compris entre 30 et 70, avantageusement entre 30 et 40, et
(b) récupération du résidu comprenant au moins 50% de DHA par rapport aux acides gras totaux du résidu ou à leurs dérivés, le rendement en DHA étant supérieur ou égal à 60%.
Sauf autrement indiqué les pourcentages en DHA (ou EPA) par rapport aux acides gras totaux ou à leurs dérivés sont exprimés en pourcentage de surface. En effet, ces valeurs correspondent à l'aire du pic attribué au DHA (ou EPA) divisée par la somme du toutes les aires de pic attribuées aux acides gras présents dans la solution ou le résidu. Ces pics sont obtenus par chromatographie en phase gazeuse selon la méthode indiquée dans la partie expérimentale et leur aire est mesurée sur les chromatogrammes obtenus.

Au sens de la présente invention on entend par « dérivés d'acide gras » les esters d'acides gras, en particulier les esters méthyliques ou éthyliques. Avantageusement il s'agit d'esters éthyliques.
De façon avantageuse, la solution d'acides gras ou de ses dérivés utilisée est une solution brute, c'est à dire n'ayant pas subi de prétraitement à l'urée, les acides gras et ses dérivés n'ayant donc pas été complexés par l'urée. Avantageusement il s'agit d'une solution d'esters éthyliques.
Avantageusement la solution d'acides gras ou de ses dérivés utilisée comprend au moins 20 %, de façon avantageuse au moins 25% de DHA par rapport aux acides gras totaux de la solution ou à leurs dérivés.
Dans un mode de réalisation particulier de l'invention, la solution d'acides gras ou de ses dérivés utilisée est une huile de poisson ou d'algue, de façon avantageuse une huile de poisson. En particulier il s'agit d'une huile de thon ou de foie de morue.

En particulier cette huile comprend au moins 20 %, de façon avantageuse au moins 25% de DHA par rapport aux acides gras totaux de l'huile ou à leurs dérivés. En particulier l'huile de foie de morue utilisée a la composition suivante, en ce qui concerne ses acides gras principaux :

| **Acide gras** | **Huile de foie de morue (%)** |
|---|---|
| 14 : 0 | 1,1 |
| 16 : 0 | 18,5 |
| 16 : 1 | 3,7 |
| 18 : 0 | 5,3 |
| 18 : 1 | 14,7 |
| 18 : 2 ω6 | 1,7 |
| 20 : 1 | 9,8 |
| 20 : 4 ω6 | 0,4 |
| 20 : 5 ω3 (EPA) | 6,4 |
| 22 : 1 | 2,3 |
| 22 : 6 ω3 (DHA) | 27,4 |
| Total saturé | 29,1 |
| Total mono insaturé | 29,7 |
| Total ω6 (LA+AA) | 2,1 |
| Total ω3 (EPA+DHA) | 33,8 |

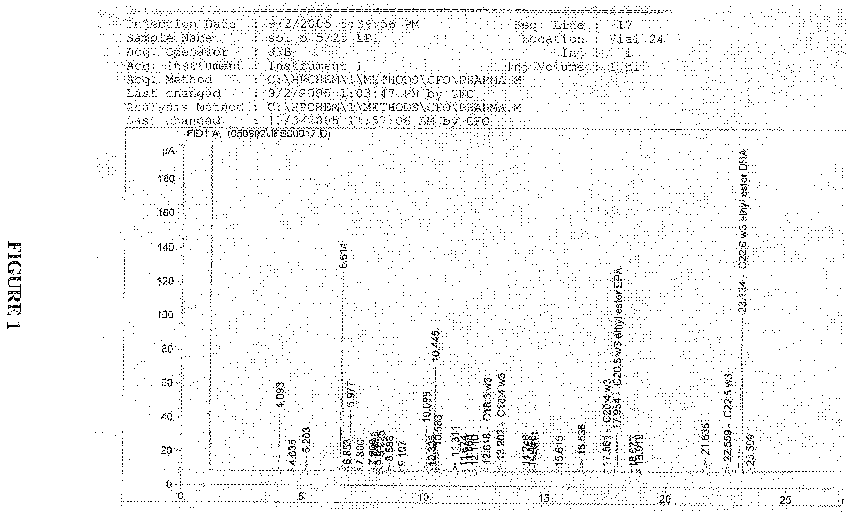

Avantageusement l'huile de thon utilisée comprend 5,5 % d'EPA et 26,6% de DHA. De façon avantageuse son chromatogramme obtenu par chromatographie en phase gazeuse par la méthode indiquée ci-après dans les exemples est représenté à la figure 1.
De façon avantageuse, il s'agit de l'huile de thon 25 DHA commercialisée par la société POLARIS ou PRONOVA BIOCARE.

Au sens de la présente invention on entend par « taux de CO₂ supercritique » le rapport du débit de CO₂ supercritique sur le débit de solution d'acides gras ou de ses dérivés.

Le procédé selon la présente invention consiste donc à injecter simultanément dans une colonne de fractionnement, à contre-courant, la solution d'acides gras ou de ses dérivés et le CO₂ supercritique. Ce dernier est injecté en pied de colonne, tandis que la solution d'acides gras ou de ses dérivés est injectée plus haut dans la colonne. Le flux de CO₂ se charge progressivement en composés les plus volatils qu'il extrait. Ces composés sont récupérés après dépressurisation dans des séparateurs cycloniques.

Dans un mode de réalisation particulier de l'invention, la température en pied de colonne est inférieure à la température en tête de colonne. Il y a donc un gradient de température au sein de la colonne. Avantageusement la température en tête de colonne est comprise entre 55 et 70°C et la température en pied de colonne est comprise entre 35 et 50°C.
La présence de ce gradient diminue la productivité du procédé mais augmente le rendement en DHA. En effet le gradient de température provoque un reflux interne ce qui peut générer un engorgement de la colonne. Il est donc nécessaire de travailler avec des débits en solution plus bas.

Dans un autre mode de réalisation de l'invention, la température de l'étape (a) est constante au sein de la colonne. Il n'y a donc pas de gradient de température. Avantageusement la température est comprise entre 40 et 70°C. L'absence de ce gradient améliore la productivité mais diminue le rendement en DHA.

Dans un mode de réalisation avantageux, la colonne de fractionnement a une longueur d'au moins 1,5 m et un diamètre interne d'au moins 20 mm, avantageusement une longueur d'au moins 8m et un diamètre interne d'au moins 126 mm.

Dans un autre mode de réalisation, la pression utilisée dans la colonne est de 135*10⁵ Pa.
Le procédé selon la présente invention permet donc d'obtenir un résidu comprenant au moins 50% de DHA par rapport aux acides gras totaux du résidu ou à leurs dérivés, avantageusement entre 50 et 70% de DHA. Ce résidu est soutiré en continu en bas de colonne. Avantageusement ce résidu comprend au moins 52% de DHA, de façon avantageuse au moins 55% de DHA, de façon encore plus avantageuse au moins 60% de DHA par rapport aux acides gras totaux du résidu ou à leurs dérivés. De façon avantageuse, le rendement en DHA du procédé selon la présente invention est d'au moins 60%, de façon avantageuse d'au moins 65%, de façon encore plus avantageuse, d'au moins 70%. Dans le cas d'un gradient de température dans la colonne, ce rendement est avantageusement d'au moins 80%.

L'invention sera mieux comprise au vu des figures et des exemples qui suivent.
La figure 1 représente un chromatogramme obtenu par chromatographie en phase gazeuse de l'huile de thon utilisée dans les exemples.

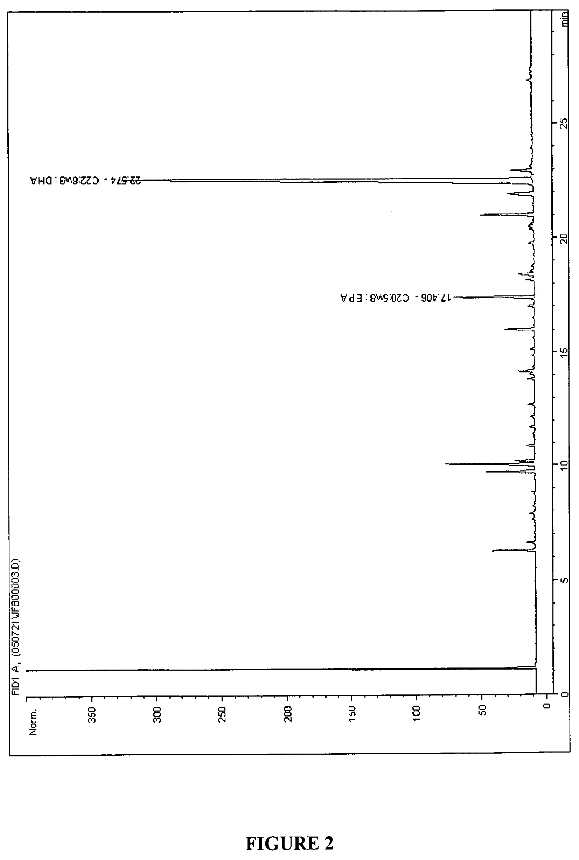

La figure 2 représente un chromatogramme obtenu par chromatographie en phase gazeuse sur le résidu enrichi obtenue à l'exemple 1.

Le protocole d'analyse des solutions enrichies et de l'huile de thon de départ est le suivant :
La composition des solutions enrichies et de l'huile de thon de départ est réalisée par chromatographie en phase gazeuse, selon la méthode suivante :
   Appareillage : - Chromatographe en Phase Gazeuse avec détecteur à ionisation de flamme

### Conditions Opératoires :

| | | | |
|---|---|---|---|
| Colonne | Type : | | CP-WAX 52CB |
| | Longueur (m) : | | 25 |
| | Diamètre (mm) : | | 0,25 |
| | | | 0,20 |
| Gaz | Gaz vecteur : | | Hélium |
| | Pression tête de colonne : | | 1,38*10⁵ Pa |
| | Mode pression ou débit constant : | | débit constant |
| | Débit colonne (ml/min) : | | 1,4 |
| Injecteur | Température (°C) : | | 250 |
| | Débit de Fuite (split) (ml/min) : | | 280 |
| | Type liner (désactivé/non désactivé) : | | désactivé |
| | Volume liner (µl): | | 860 |
| Détecteur | Température (°C) : | | 270 |
| | Débit Hydrogène (ml/min) : | | 30 |
| | Débit Oxygène (ml/min) : | | 300 |
| | Make-up + colonne (ml/min) : | | 30 |
| Programme du four | Rampe (°C/min) | Température (°C) | Durée (min) |
| | | 170 | 2 |
| | 2,95 | 240 | 2,30 |
| Durée de l'analyse (min) | 28,03 | | |
| Volume d'injection (µl) | 1 | | |

### Méthodologie :

### Préparation d'une solution témoin :

Dans une fiole de 5,0 ml, introduire 30 mg d'ester éthylique d'acide docosahexaénoïque SCR (DHA), 45 mg d'ester éthylique d'acide éicosapentaénoïque SCR (EPA). Dissoudre dans une solution de butylhydroxytoluène R à 50 mg/l dans le triméthylpentane R et compléter au volume avec la même solution.

### Préparation de la solution à analyser (2 essais) :

Dans une fiole de 10,0 ml, introduire 0,5 g de substance à examiner .Dissoudre dans une solution de butylhydroxytoluène R à 50 mg/l dans le triméthylpentane R et compléter au volume avec la même solution.

### Exploitation des résultats :

Relever les % surfaces de l'ester éthylique d'acide éicosapentaénoïque (EPA) et de l'ester éthylique d'acide docosahexaénoïque (DHA) dans la solution à analyser.

Les exemples suivants sont donnés à titre indicatif non limitatif.

### Exemple 1 :

Sur une colonne de laboratoire garnie de 1,5 m de haut et de diamètre interne de 20mm, on injecte 1520 g d'huile de thon 25 DHA commercialisée par la société POLARIS dont le chromatogramme est représenté à la figure 1 et contenant 5,5 % d'EPA et 26,6% de DHA. La durée totale de l'injection est de 4 heures.
Les paramètres opératoires sont les suivants :
- Pression et température dans la colonne : 135*10⁵ Pa - 60°C.
- Débit d'injection de la solution : 280 g/h.
- Débit de CO₂ : 19 kg/h.
Le taux de CO₂ supercritique est donc de 67,8.
On soutire en continu en pied de colonne la solution enrichie. On récupère ainsi 534g d'une solution d'esters éthyliques d'acides gras contenant 55,0% de DHA par rapport aux acides gras totaux ou à leurs dérivés. Le rendement de récupération du DHA est de 77%.

### Exemple 2 :

Sur une colonne garnie industrielle de 8 m de haut et de diamètre interne de 126mm, on injecte 210,6 kg d'huile de thon 25 DHA commercialisée par la société POLARIS dont le chromatogramme est représenté à la figure 1 et contenant 5,5 % d'EPA et 26,6% de DHA. La durée totale de l'injection est de 9 heures.
Les paramètres opératoires sont les suivants :
- Pression et température dans la colonne : 135*10⁵ Pa - 60°C.
- Débit d'injection de la solution : 23 kg/h.
- Débit de CO₂ : 900 kg/h.
Le taux de CO₂ supercritique est donc de 39,1.
On soutire en continu en pied de colonne la solution enrichie. On récupère ainsi 63,3 kg d'une solution d'esters éthyliques d'acides gras contenant 63,7% de DHA par rapport aux acides gras totaux ou à leurs dérivés. Le rendement de récupération du DHA est de 64%.

### Exemple 3 :

Sur une colonne garnie industrielle de 8 m de haut et de diamètre interne de 126mm, on injecte 203,0 kg d'huile de thon 25 DHA commercialisée par la société POLARIS dont le chromatogramme est représenté à la figure 1 et contenant 5,5 % d'EPA et 26,6% de DHA. La durée totale de l'injection est de 8,5 heures.
Les paramètres opératoires sont les suivants :
- Pression et température dans la colonne : 135*10⁵ Pa - 60°C.
- Débit d'injection de la solution : 24 kg/h.
- Débit de CO₂ : 800 kg/h.
Le taux de CO₂ supercritique est donc de 33,3.
On soutire en continu en pied de colonne la solution enrichie. On récupère ainsi 85,4 kg d'une solution d'esters éthyliques d'acides gras contenant 53,5% de DHA par rapport aux acides gras totaux ou à leurs dérivés. Le rendement de récupération du DHA est de 75%.

### Exemple 4 :

Sur une colonne de laboratoire garnie de 1,5 m de haut et de diamètre interne de 20mm, on injecte 333 g d'huile de thon 25 DHA commercialisée par la société POLARIS dont le chromatogramme est représenté à la figure 1 et contenant 5,5 % d'EPA et 26,6% de DHA. La durée totale de l'injection est de 1 heure 45.
Les paramètres opératoires sont les suivants :
- Pression dans la colonne : 135*10⁵ Pa.
- Gradient de température dans la colonne : 45°C en pied de colonne et 65°C en tête de colonne.
- Débit d'injection de la solution : 220 g/h.
- Débit de CO₂ : 15 kg/h.
Le taux de CO₂ supercritique est donc de 68.
On soutire en continu en pied de colonne la solution enrichie. On récupère ainsi 166 g d'une solution d'esters éthyliques d'acides gras contenant environ 50% de DHA par rapport aux acides gras totaux ou à leurs dérivés. Le rendement de récupération du DHA est de 88% et la productivité de 95g/h.

### Exemple 5 :

Sur une colonne de laboratoire garnie de 1,5 m de haut et de diamètre interne de 20mm, on injecte 800 g d'huile de thon 25 DHA commercialisée par la société POLARIS dont le chromatogramme est représenté à la figure 1 et contenant 5,5 % d'EPA et 26,6% de DHA. La durée totale de l'injection est de 1h50.
Les paramètres opératoires sont les suivants :
- Pression et température dans la colonne : 135*10⁵ Pa - 60°C.
- Débit d'injection de la solution : 440 g/h.
- Débit de CO₂ : 29 kg/h.
Le taux de CO₂ supercritique est donc de 65,9.
On soutire en continu en pied de colonne la solution enrichie. On récupère ainsi 272g d'une solution d'esters éthyliques d'acides gras contenant 51,0% de DHA par rapport aux acides gras totaux ou à leurs dérivés. Le rendement de récupération du DHA est de 65%. La productivité est de 160g/h.

## Revendications

1. Procédé continu d'enrichissement en DHA d'une solution d'acides gras ou de ses dérivés comprenant moins de 50% de DHA par rapport aux acides gras totaux de la solution ou à leurs dérivés, le procédé comprenant les étapes de
(a)- injection simultanée à contre-courant dans une colonne de fractionnement du flux de la solution d'acides gras ou de ses dérivés et d'un flux de CO₂ supercritique à une température inférieure ou égale à 70°C et à une pression comprise entre 100*10⁵ Pa et 160 *10⁵ Pa, le taux en CO₂ supercritique étant compris entre 30 et 70 et
(b) récupération du résidu comprenant au moins 50% de DHA par rapport aux acides gras totaux du résidu ou à leurs dérivés, le rendement en DHA étant supérieur ou égal à 60%.

2. Procédé selon la revendication 1 **caractérisé en ce que** la température en pied de colonne est inférieure à la température en tête de colonne, avantageusement la température en tête de colonne est comprise entre 55 et 70°C et la température en pied de colonne est comprise entre 35 et 50°C.

3. Procédé selon la revendication 1 **caractérisé en ce que** la température de l'étape (a) est constante au sein de la colonne, avantageusement est comprise entre 40 et 70°C.

4. Procédé selon l'une quelconque des revendications précédentes **caractérisé en ce que** l'étape (a) est réalisée à une pression comprise entre 120*10⁵ Pa et 140*10⁵ Pa.

5. Procédé selon l'une quelconque des revendications précédentes **caractérisé en ce que** la solution d'acides gras ou de leurs dérivés est une solution d'huile de poisson, avantageusement d'huile de thon ou de foie de morue.

6. Procédé selon la revendication 5 **caractérisé en ce que** la solution d'huile de poisson comprend au moins 20% de DHA par rapport aux acides gras totaux de la solution d'huile de poisson ou à leurs dérivés.

7. Procédé selon l'une quelconque des revendications précédentes **caractérisé en ce que** la solution d'acides gras ou de ses dérivés est une solution d'esters éthyliques d'acides gras.

8. Procédé selon l'une quelconque des revendications précédentes **caractérisé en ce que** le taux de CO₂ supercritique de l'étape (a) est compris entre 30 et 40.

## Patentansprüche

1. Kontinuierliches Verfahren zur Anreicherung mit DHA einer Lösung aus Fettsäuren oder ihren Derivaten, umfassend mindestens 50 % DHA mit Bezug auf die gesamten Fettsäuren der Lösung oder ihre Derivate, wobei das Verfahren die Folgenden Schritte umfasst:
(a) gleichzeitiges Injizieren gegen den Strom in eine Fraktionierungssäule des Flusses der Lösung von Fettsäuren oder ihren Derivaten und eines Flusses von superkritischem CO₂ bei einer Temperatur von weniger als oder gleich wie 70 °C und bei einem Druck, der zwischen 100*10⁵ Pa und 160*10⁵ Pa liegt, wobei die Rate von superkritischem CO₂ zwischen 30 und 70 liegt, und
(b) Wiedergewinnen des Rückstands, umfassend mindestens 50 % DHA mit Bezug auf die gesamten Fettsäuren des Rückstands oder ihre Derivate, wobei die Ausbeute von DHA größer als oder gleich wie 60 % ist.

2. Verfahren nach Anspruch 1, **dadurch gekennzeichnet, dass** die Temperatur am Fuß der Säule niedriger als die Temperatur am Kopf der Säule ist, vorzugsweise liegt die Temperatur am Kopf der Säule zwischen 55 und 70 °C, und die Temperatur am Fuß der Säule liegt zwischen 35 und 50 °C

3. Verfahren nach Anspruch 1, **dadurch gekennzeichnet, dass** die Temperatur von Schritt (a) in der Säule konstant ist, vorteilhafterweise zwischen 40 und 70 °C liegt.

4. Verfahren nach einem der vorhergehenden Ansprüche, **dadurch gekennzeichnet, dass** der Schritt (a) bei einem Druck durchgeführt wird, der zwischen 120*10⁵ Pa et 140*10⁵ Pa liegt.

5. Verfahren nach einem der vorhergehenden Ansprüche, **dadurch gekennzeichnet, dass** die Lösung von Fettsäuren oder ihren Derivaten eine Lösung aus Fischöl, vorteilhafterweise aus Thunfisch oder aus Lebertran ist.

6. Verfahren nach Anspruch 5, **dadurch gekennzeichnet, dass** die Lösung aus Fischöl mindestens 20 % DHA mit Bezug auf die gesamten Fettsäuren der Lösung aus Fischöl oder ihrer Derivate umfasst.

7. Verfahren nach einem der vorhergehenden Ansprüche, **dadurch gekennzeichnet, dass** die Lösung aus Fettsäuren oder ihrer Derivate eine Lösung aus Ethylethern von Fettsäuren ist.

8. Verfahren nach einem der vorhergehenden Ansprüche, **dadurch gekennzeichnet, dass** die Rate von superkritischem CO₂ von Schritt (a) zwischen 30 und 40 liegt.

## Claims

1. Continuous process of DHA enrichment of a solution of fatty acids or of derivatives thereof comprising less than 50% DHA relative to the total fatty acids of the solution or to the derivatives thereof, wherein the process comprises the steps of
(a) simultaneous countercurrent injection, into a fractionating column of the flow of the solution of fatty acids or of derivatives thereof and of a flow of supercritical CO₂ at a temperature less than or equal to 70°C and at a pressure between 100*10⁵ Pa and 160*10⁵ Pa, wherein the rate of supercritical CO₂ is between 30 and 70 and
(b) recovery of the residue comprising at least 50% DHA relative to the total fatty acids of the residue or to derivatives thereof, wherein the DHA yield is greater than or equal to 60%.

2. Process according to claim 1 **characterised in that** the temperature at the column bottom is less than the temperature at the column head, advantageously, the temperature at the column head is between 55 and 70°C and the temperature at the column bottom is between 35 and 50°C.

3. Process according to claim 1 **characterised in that** the temperature of step (a) is constant in the column, and advantageously is between 40 and 70°C.

4. Process according to any of the preceding claims **characterised in that** step (a) is performed at a pressure between 120*10⁵ Pa and 140*10⁵ Pa.

5. Process according to any of the preceding claims **characterised in that** the solution of fatty acids or of derivatives thereof is a solution of fish oil, advantageously tuna or cod liver oil.

6. Process according to claim 5 **characterised in that** the fish oil solution comprises at least 20% DHA relative to the total fatty acids of the fish oil solution or to derivatives thereof.

7. Process according to any of the preceding claims **characterised in that** the solution of fatty acids or of derivatives thereof is a fatty acid ethyl ester solution.

8. Process according to any of the preceding claims **characterised in that** the rate of supercritical CO₂ of step (a) is between 30 and 40.
